# EUROPEAN PATENT SPECIFICATION

(11) **EP 1 841 476 B1**
(45) Date of publication and mention of the grant of the patent: **29.06.2011**
(21) Application number: 06707708.1
(22) Date of filing: 12.01.2006
(51) Int. Cl.: A61M 5/142, A61M 5/172, A61B 5/0402

(54) **FLUID DELIVERY DEVICE WITH INTEGRATED MONITORING OF PHYSIOLOGICAL CHARACTERISTICS**
FLÜSSIGKEITSAUSGABEVORRICHTUNG MIT INTEGRIERTER ÜBERWACHUNG VON PHYSIOLOGISCHEN EIGENSCHAFTEN
DISPOSITIF DE DISTRIBUTION DE FLUIDE A CONTROLE INTEGRE DE CARACTERISTIQUES PHYSIOLOGIQUES

(30) Priority: 17.01.2005 DK 200500081
(43) Date of publication of application: 10.10.2007
(73) Proprietor: NOVO NORDISK A/S, 2880 Bagsvaerd (DK)
(72) Inventor: BENGTSSON, Henrik, DK-2000 Frederiksberg (DK); KRISTENSEN, Leif, Engmann, DK-2000 Frederiksberg (DK); LEVANDIS, Nicola, DK-9100 Aalborg (DK); STRUIJK, Johannes, Jan, DK-9575 Terndrup (DK); LE TOMMY, 9000 Aalborg (DK); RANDLOEV JETTE, 3500 Vaerloese (DK)
(86) International application number: PCT/EP2006/050176
(87) International publication number: WO 2006/075016

(56) References cited:
- WO-A-88/05282
- WO-A-02/069798
- WO-A-03/092487
- US-A- 5 050 612
- US-A- 5 527 288
- US-A- 5 741 211

## Description

The present invention relates generally to systems for managing medical therapy. More specifically, the invention relates to drug delivery devices incorporating means for detecting the presence or onset of a physiological condition, such as hypoglycemia, in a patient.

### BACKGROUND OF THE INVENTION

In the disclosure of the present invention reference is mostly made to the treatment of diabetes by injection of insulin, however, this is only a preferred use of the present invention.

Diabetes mellitus is the common name for at least 2 different diseases, one characterised by immune system mediated specific pancreatic beta cell destruction (insulin dependent diabetes mellitus (IDDM) or type 1 diabetes), and another characterised by decreased insulin sensitivity (insulin resistance) and/or a functional defect in beta cell function (non-insulin dependent diabetes mellitus (NIDDM) or type 2 diabetes).

The principal treatment of type 1 diabetes is straight forward substitution of the missing insulin secretion, whereas treatment of type 2 is more complicated. More specifically, in early stages of type 2 diabetes treatment a number of different types of drugs can be used, e.g. drugs which increase insulin sensitivity (ciglitazones), decrease hepatic glucose output (e.g. metformin), or reduce glucose uptake from the gut (alfa glucosidase inhibitors), as well as drugs which stimulate beta cell activity (e.g. sulfonylurea/meglitinides). However, the above-described deterioration is reflected in the fact that beta cell stimulators will eventually fail to stimulate the cell, and the patient has to be treated with insulin, either as mono therapy, or in combination with oral medication in order to improve glucose control.

Currently, there are two principal modes of daily insulin therapy, the first mode including syringes and insulin injection pens. These devices are simple to use and are relatively low in cost, but they require a needle stick at each injection, typically 3-4 times or more per day. The second mode is infusion pump therapy, which entails the purchase of a portable but relatively expensive pump, for which reason the initial cost of the pump is a barrier to this type of therapy. Although more complex than syringes and pens, the pump offer the advantages of continuous infusion of insulin, precision in dosing and optionally programmable delivery profiles and user actuated bolus infusions in connections with meals. Further, in combination with a blood glucose sensor an infusion pump may provide fully automatic closed loop control of insulin infusion.

Recently less expensive infusion pumps have been proposed which may either be fully disposable providing only the most basic functions such as a constant basal rate, or infusion pump systems comprising a disposable portion in combination with a durable control portion, where the latter may provide many of the more advanced features of the traditional pump.

In order to maintain a proper dosing of insulin, whether administered with an insulin injecting pen or with an infusion pump, most diabetic patients are required more or less frequently to monitor the blood glucose in order to maintain an acceptable treatment with insulin. Monitoring of blood glucose presently requires the use of glucose meters which typically relies on small blood samples taken with a lancet. For most users, frequent measurements of blood glucose on the basis of blood samples are painful and not suited for continuous monitoring.

Conventional glucose monitoring systems are somewhat limited in the feature that they provide to facilitate the monitoring of blood glucose levels. Typically, a glucose monitor will take readings as directed by the user and might provide a warning if a reading is deemed at an unsafe level (e.g., a hyper- or hypoglycemic condition). However, by the time the warning occurs, the user may already be experiencing negative symptoms.

Although continuous glucose monitoring systems using transcutaneous or subcutaneous implanted glucose sensors have been proposed, these systems still require invasive insertion of the sensors. Furthermore, the CGM systems thus far presented are not always sufficiently reliable as the sensors suffer from stability problems.

One of the most critical complications experienced by patients suffering from insulin dependant diabetes mellitus is the phenomenon of hypoglycemia. Hypoglycemia is a physiological condition where the blood glucose level of the patient decreases below a certain value. Blood glucose levels below approx. 2.5mmol/L may give rise to serious symptoms and may potentially even become dangerous for a diabetic patient, in particular, if the patient does not become aware of the condition, e.g. because the patient is sleeping or preoccupied with another activity, e.g. driving a car.

As is known in the art, a glucose crash occurs when blood glucose levels of an individual are in a state of rapid decline and its symptoms are similar to hypoglycemia. The symptoms are caused by the dynamics of a declining glucose level and not by an absolute glucose level.

Already during the onset of hypoglycemia more moderate drops of the blood glucose level, e.g. below approximately 3.8 mmol/L glucagon, cause epinephrine, growth hormone, and cortisol to be released, resulting in symptoms such as rise in pulse, lowering of the variability of the heart rate and increased perspiration.

Both due to the risks of reaching unhealthy or even fatal physiological conditions, and due to the desire to be able to normalize the glucemic level in the body, there is a long felt need to be able to monitor the physiological conditions of a patient on a continuously basis.

Different non-invasive sensors have been proposed for obtaining a measure of blood glucose constituents in blood, e.g. using infrared BGM. Also, US Patent No. 5,741,211 describes a system for providing an indication of either blood insulin or blood glucose in a patient, where the indication is based on correlations between changes in various ECG parameters and the level of the particular blood constituents. The indications are either communicated to the patient for manual control of an insulin pump (open loop control) or used to provide a closed loop system for automatically providing insulin to the patient. The system is described as either a wearable pack, which has connections to conventional electrodes for detecting the ECG parameters, or as an implantable apparatus having a lead which picks up patient intracardiac or epicardiac signals. The sensing of the ECG parameters is disclosed as conventional ECG sensors such as implanted leads, ECG electrodes in the form of skin surface electrodes or subcutaneous electrodes, all of which are connected to the monitoring device by means of electrical wirings.

An alternative approach to detection of an absolute level of glucose plasma in the body of a patient is monitoring the state of different characteristic physiological parameters which correspond to one or more specific physiological conditions of the patient. For example, a patient being at the onset or experiencing a hypoglycemic state show various different symptoms such as a change in skin impedance arising from increased perspiration, reduced body temperature as well as alterations in the ECG, i.e. T-wave is flattened, QT interval is prolonged, the heart rate increases as well as a lowering of the variability of the heart rate, etc. WO Patent application No. 02/069798 discloses a method for determining the onset or presence of a physiological condition such as a hypoglycemia by monitoring various physiological parameters such as the person's skin impedance, skin temperature, pulse, respiration data, CO₂ content of the blood, ECG related signals such as heart rate variability, QT interval and EEG such as the mean or peak frequency of the α wave.

ECG monitoring using surface-mounted ECG-sensors is usually conducted using skin mounted ECG electrodes attached to the skin at the chest region of the patient. This arrangement provides optimal conditions for picking up heart signals due to the small distance from the heart.

Apart from systems using a number of separate ECG electrodes each mounted to adjacent regions of the patient, US Patent No. 6,775,566 discloses a band-like ECG sensor arrangement attached to the skin of a person's chest for measuring electrical heart beat signals. US Patent No. 4,129,125 discloses a related arrangement comprising an elastic belt or strap which is designed to fit around the patient's chest or stomach area. As both of these systems rely on sensors being arranged in a band-like structure applied to the skin along a relatively long stretch, patient comfort is somewhat compromised mainly due to chest or abdominal region movements caused by patient respiration or physical activity.

A further system for monitoring physiological conditions is shown in WO Patent application No. 88/05282 which describe a portable, self-contained, physiological monitor which is applied to the chest of a user. The monitor case comprises integral sensors arranged to protrude from the case for application to the skin of the user. The integral sensors comprise ECG electrodes which may be combined with sound and temperature sensors. While addressing the object of integration of electrodes, sensing circuitry, data processing means and output means into a single housing, the user still has to wear one or more additional devices for control of the medication to be delivered.

The same considerations apply to the drug delivery device of US Patent No. 6,749,587 which comprises a data communication assembly for wireless communication with an implanted or external sensor or device, transferring diagnostic data such as an electrocardiogram to the data communication assembly.

One particular patch pump for delivering a drug to a patient is shown in US Patent No. 5,527,288, which discloses a skin mountable pump comprising a sensor providing a feedback arrangement for detecting a condition in the body of the subject and for controlling the delivery of the drug in response thereto. The sensor may be a temperature sensor, a pulse rate sensor, a blood glucose sensor, a blood pressure sensor or a PH sensor. The sensor may rest against the skin, may be inserted through the skin, or may be within the device and separate from the skin. By only using a single sensor, the degree of sensing conditions is limited to somewhat simple routines making extensive analysis unreliable or even impossible.

Infusion pumps which provide a continuous or intermittent supply of a specific drug are usually carried at the abdominal region in order to accomplish a consistent rate of absorption of medication. This goes hand in hand with most users desire to hide the pump under clothing so as not to seem different from non-diabetic people. As discussed above, according to the prior art, monitoring physiological conditions on the basis of ECG-related parameters necessitates either a second dedicated monitor for sensing the ECG signals or additional electrical connections to external sensor electrodes.

When using an infusion pump, it would be desirable to be able to monitor characteristic physiological conditions or parameters on a continuous or intermittent basis without the need to rely on external monitoring devices or external leads connected to the pump.

One particular device offering the combination of a delivery device and ECG monitoring is disclosed in WO Patent application No. 03/092487 which shows a medical doser. In this document, electrodes for obtaining ECG signals are provided on the engagement face of the medical doser with a further electrode arranged on the handle of the doser. In this configuration, during injection, the electrodes on the engaging face is applied to the skin of the user, while the user creates a signal path by gripping the handle of the doser with a hand, thus providing a signal path through the user's heart region. However, since this device is only applied to the body at the user's option, this device is not practical for full-time monitoring. In particular, this applies especially during sleep.

### DISCLOSURE OF THE INVENTION

Having regard to the above-identified problems and deficiencies, it is an object of the present invention to provide a fluid delivery device which in its basic form comprises a single portable item and which effectively and reliable can be used to monitor physiological data recorded within said medication delivery device. It is a further object of the invention to provide a fluid delivery device, which can be used to monitor the onset or presence of a physiological condition of a user.

More specifically, the invention is based on the concept that ECG related signals, originating from a user carrying the delivery device, can be picked up by ECG sensor elements arranged on or in the delivery device itself, thereby facilitating recording or processing of ECG related signals internally in said device, and hence, offer a response to a detected physiological condition from said ECG related signals.

Delivery patches incorporating ECG electrodes and ECG preamplifiers per se are well known in the medical art for the sensing of therapeutical electrical outputs from implantable devices like pacemakers or implantable cardioverter defibrillators (ICD's). As indicated, these patches rely on an implanted device which provokes a therapeutical output. Such systems are known from WO Patent application No. 02/87681.

However, the drug delivery device of the present invention is intended to encompass drug delivery devices which are able to sense ECG related electrical signals which are naturally occurring inside the subject user i.e. without the use of artificially provoked therapeutical electrical outputs. In this application such naturally occurring signals are defined as native electrical signals.

According to the present invention a fluid delivery device as defined in claim 1 is provided.

Thus, in a first aspect the present a fluid (e.g. drug) delivery device comprises a housing for attachment to the skin of a user where the device comprises a reservoir adapted to contain a liquid drug and, in a situation of use, associated outlet means, as well as expelling means for expelling a drug out of the reservoir through the outlet means. The device further comprises a voltage and energy source and ECG sensor elements arranged on or in the housing of the drug delivery device so as to be, in a situation of use, in close proximity of the skin of the user for picking up ECG related signals originating from said user. The ECG sensor elements are preferably connected to dedicated electric circuitry for recording the sensed ECG signals either partial or in full.

The term "housing" merely denotes a supporting structure for supporting the different elements as described. The housing may be a traditional partially or fully closed structure. However, it may also be in the form of an open structure, e.g. a platform.

The definition that the ECG sensor elements are arranged on or in housing of the drug delivery device should be construed as including embodiments where the drug delivery device and one or more of the sensor elements are arranged so as be one self-contained unit, i.e. having one or more electrodes integral with the delivery device. However, said one or more electrodes may be arranged so as to, in a situation of use, extend partially beyond the perimeter of the housing of the drug delivery device. The definition furthermore should be construed to include embodiments where at least one electrode is arranged completely or partly between the mounting surface of the housing and the skin of the user when the delivery device is affixed to the user. For a user, such a configuration will be conceived as wearing a single entity.

The sensed ECG signals which is recorded or transmitted may contain information regarding parameters such as heart rate, heart rate variability (HRV), QT interval, QT dispersion, polarity of T-wave, amplitude of T-wave, T-wave ratio, QRS amplitude, QRS length etc. The electronic circuitry may be designed to sense one or more of the parameters mentioned above. Also, the respiration rate may be extracted from the recorded ECG signals.

In a second aspect, the delivery device may be provided with additional sensor inputs providing additional information of other physiological or physical parameters to assist in the analysis or decision support. Such sensor inputs may be provided by the same sensor elements as the ones used for ECG signal pick-up, or may be provided by additional sensors arranged within or on the housing of the delivery device. Additional sensors may also be arranged separate from the housing of the drug delivery device communicating with the delivery device either by wired connection or wireless connection. While the delivery device in its most basic form only contains sensor elements integrally with the housing, such additional separate sensors may, at specific situations such as during extraordinary physical activity, be attached to other parts of the user's body. Such additional sensors may be adapted to detect other parameters which may include galvanic skin resistance, skin impedance spectra, electromyograms (EMG), pulse rate, skin temperature, ambient temperature, oxygen saturation, blood pressure, lung wetness, glucose level, thoracic impedance, ICV pressure, CO₂ content of blood, EEG, mean or peak frequency of the alpha wave, position of patient, movement of body, pH-value, respiration rate, acoustic sounds of the heart picked up by a microphone or may be adapted to obtain any other parameter sensed by electric, electromagnetic, acoustic or optic means.

In an exemplary embodiment, the drug delivery device may further be provided with an observable indicator based upon the recorded signals, or alternatively, the device may be provided with a storage facility for analyzing the received signals retrospectively for example by downloading the stored signals to an external device for processing and analyzing the recorded parameters. Such data transmittal may include transmittal of partial or full representations of the sensed ECG signals. In this configuration the drug delivery device may be used as a monitoring tool for the patient to get acquainted with bodily reactions of different treatments schemes or unfamiliar physical activity levels. Alternatively, it can be used as an evaluation tool under a physician's supervision. The drug delivery device can also be designed for continuously or intermittently communicating data related to sensed parameters to an external device for real-time analyzing data in a portable external device.

The drug delivery device may be provided with specific electronic circuitry such as a microprocessor for analyzing the sensed sensor parameters in order to provide data indicative of the presence or the onset of a physiological condition of the user. The electronic circuitry may include a DSP or decision system for analyzing the recorded signals, extracting features of interest, and comparing the extracted features with predefined data for identifying one or more physiological patient conditions.

As far as detecting physiological conditions, the drug delivery device may be used as a monitor providing an estimate of the overall physical activity of the user, based on the physiological measurements, for example heart rate, temperature, EMG level, body movements obtained by an accelerometer etc. An activity level monitor according to the invention provides valuable information which can be used for the prediction of the need for injecting insulin.

Also, the drug delivery device may be adapted to sense conditions which are characteristic for the onset or presence of a hypoglycemic state. In particular, it is an advantage that the delivery device automatically detects nightly hypoglycemia for diabetic patients sufficiently early for the patient to handle the problem himself/herself. The sensor signals which has been picked up by the ECG-signals is analyzed and may cause an alarm if the analysis shows that the patient experiences a hypoglycemia or is on the way to one. Likewise, other exemplary and non-limiting embodiments may include the drug delivery device adapted for sensing physiological conditions such as the onset or presence of a hyperglycemic state or sleep activity of the user.

The drug delivery device may be provided with associated output means either for presenting sensed physiological parameters or for signalling the onset or presence of a physiological condition which has been identified. The output means may provide signals selected from the group consisting of audible signals, visual signals, tactile signals, electro-muscle stimulation and vibratory signals. Also, the output means may comprise communication means for sending an alarm to an external device.

The determination of physiological conditions may be used to generate command signals in response thereto in order to regulate the drug intake. In such a configuration, the drug delivery device may be adapted to keep the blood glucose level of the patient within a desired range, either by closed loop control, or by manual intervention. The closed loop design comprises delivery means for delivering an amount of a drug having a blood glucose regulating effect, wherein operation of the delivery means is affected by the command signals.

The outlet means associated with the reservoir of the drug delivery device may be in direct fluid communication with the reservoir (e.g. in case the expelling means is arranged "before" the reservoir as for a piston pump) or indirect fluid communication (e.g. in case the expelling means is arranged "after" the reservoir as for a membrane pump). The outlet means may be adapted to be brought in fluid communication with infusion means a flexible infusion cannula. In the latter case the fluid communication may be established just prior to use, before or after the drug delivery device has been arranged on the user. Also, the transcutaneous access means may include transdermal drug delivery provided as electrotransport devices, such as iontophoresis devices.

The fluid delivery device may be intended to be fully disposable, partially disposable (i.e. with the different components of the device arranged in either a disposable or a durable portion) or durable, it may be prefilled just as it may provide constant rate infusion only or also bolus delivery. The expelling means may be of any desirable nature, such as known from US Patents 4,340,048 and 4,552,561 (based on osmotic pumps), US Patent 5,858,001 (based on a piston pump), US Patent 6,280,148 (based on a membrane pump), US Patent 5,957,895 (based on a flow restrictor pump (also know as a bleeding hole pump)), or US Patent 5,527,288 (based on a gas generating pump), which all in the last decades have been proposed for use in inexpensive, primarily disposable drug infusion pumps.

In an exemplary embodiment the drug delivery device comprises a mounting surface adapted for application directly to the skin of the user, the first sensor elements being arranged on the mounting surface which advantageously comprises adhesive means (e.g. a pressure-sensitive adhesive) which allows the device to be affixed to the skin of the subject user. Alternatively, the drug delivery device is attached to the patient using a belt or strap.

In a preferred form, the delivery device is adapted to be affixed to the abdominal region of a subject user.

In a third aspect of the invention, the abovementioned drug delivery device is configured to be used in combination with an external device, which may be a portable device, carried by the user of the drug delivery device or, alternatively, a non-portable device for stationary use. The external device is configured for receiving sensor data transferred from the drug delivery device and for storing the received data in said external device. The transferred data may comprise partial or full representations of sensed ECG related signals. Also, full or partial representations of other parameters picked up by the sensor elements of the drug delivery device may be transferred to the external device. Depending on the extent of data processing in the drug delivery device, the external device may be adapted to comprise processing means for analyzing the received data, extracting features of interest, and comparing the extracted features with predefined data for identifying one or more physiological patient conditions. The external device may hold associated output means either for presenting sensed physiological parameters or for signalling the onset or presence of a physiological condition which has been identified. The output means of the external device may provide signals selected from the group consisting of audible signals, visual signals, tactile signals, electro-muscle stimulation and vibratory signals. Also, the output means may comprise communication means for sending an alarm to a remote emergency centre. Furthermore, the output means may generate signals which are transferred to the drug delivery device for control of the timing and the amount of the drug to be delivered.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the exemplary embodiments reference will be made to the use of insulin. Correspondingly, the terms "subcutaneous" and "transcutaneous" infusion is meant to encompass any method of transcutaneous delivery to a subject.

### BRIEF DESCRIPTION OF THE DRAWINGS

In the following the invention will be further described with references to the drawings, wherein
fig. 1 a is a schematic representation of a first embodiment of the invention,
fig. 1b shows a block diagram of an exemplary sensing circuitry which is adapted for incorporation in the drug delivery device according to the invention,
fig. 2a is side view of a second embodiment of the invention,
fig. 2b is a partly sectional view along line A-A of the second embodiment shown if fig. 2a,
fig. 2c is a bottom view of the second embodiment shown in fig. 2a,
fig. 2d is a top view of the second embodiment shown if fig. 2a,
fig. 3 is a perspective view of a third embodiment where a disposable assembly and a reusable assembly of the medical device have been disassembled,
fig. 4a and b shows a side view and a bottom view of a disposable assembly of the medical device according to a fourth embodiment,
fig. 5 shows a bottom view of the mounting surface of a fifth embodiment, and
fig. 6 shows a bottom view of the mounting surface of a sixth embodiment.

### DESCRIPTION OF EXEMPLARY EMBODIMENTS

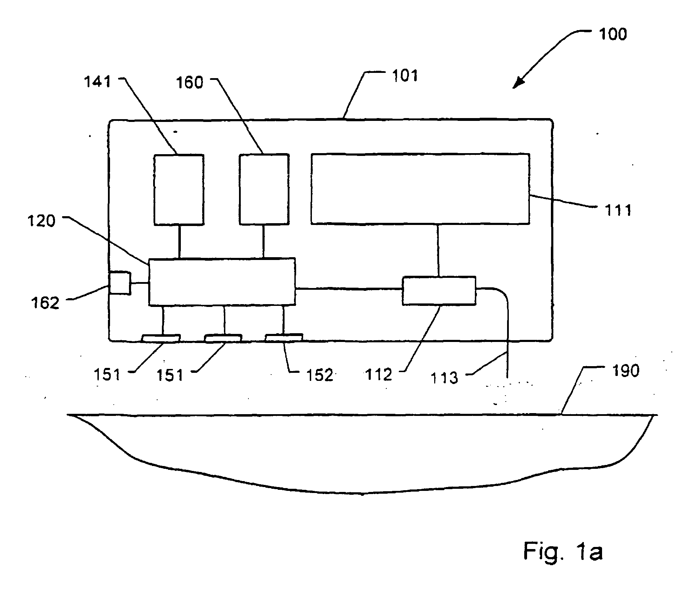

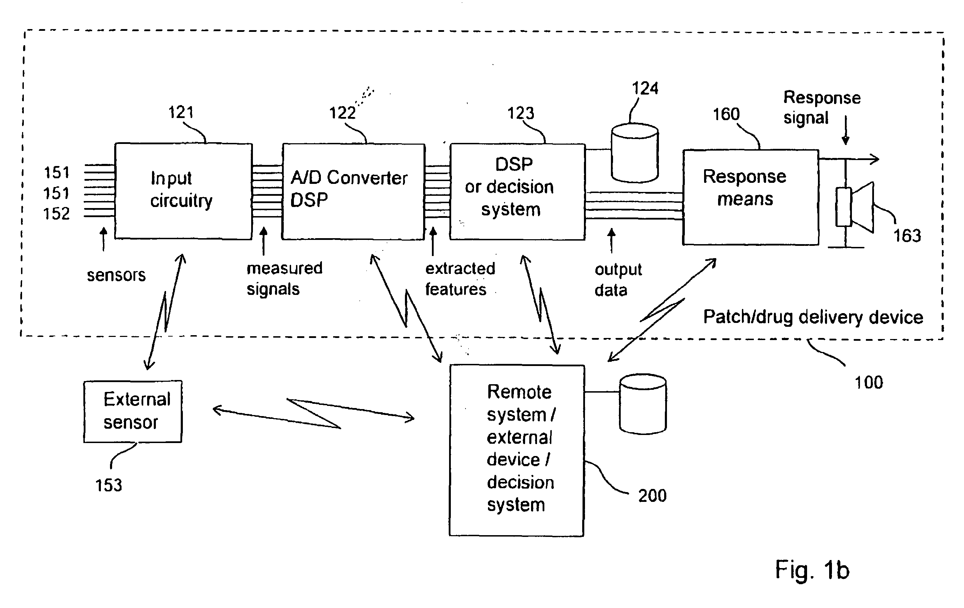

Figs. 1-5 show schematic representations of embodiments of the invention. Correspondingly, the configuration of the different structures as well as their relative dimensions and positions are intended to serve illustrative purposes only.

More specifically, fig. 1a shows a drug delivery device 100 in the form of a medical patch adapted to be worn by a user. The drug delivery device 100 comprises a housing 101 which contains a drug reservoir 111 in fluid communication with a pump 112 (e.g. a membrane pump) adapted for infusing a drug into a body of a user via infusion needle 113 in accordance with instructions received from the control means of a micro processor 120. The pump may be of the metering type, i.e. the amount of drug infused corresponds to the controlling signals received from the processor or the infusion unit may be provided with detecting means for determining the amount of drug actually infused. A voltage or energy source 141 is provided in the form of a battery supplying energy to the processor as well as the pump and detecting means (via the processor).

The drug delivery device 100 further comprises one or more first sensor elements 151 adapted to be mounted in conductive contact with the skin of a subject. The first sensor elements 151 in the form of skin contact electrodes are adapted to pick up heart related electrical signals from the patient's heart, such signals may include heart rate variability (HRV), heart rate such as measured by the RR interval, QT interval, amplitude of the T-wave, QRS amplitude, QRS length, respiration rate, etc., or any combination of the above. In addition, the first sensor elements 151 may be adapted to detect other signals which may include galvanic skin resistance, skin impedance spectra, electromyograms (EMG) or any other kind of signal which is detectable by skin contact electrodes.

The first sensor elements 151 forward the measured signals to a central processing unit or micro processor 120. In one embodiment, the signals are forwarded as analog signals to the central processing unit. In such a system, the electronics of micro processor 120 may include an ECG signal amplifier including hardware filters coupled with first sensor elements 151 for producing an analog signal representative of the electrical field on the surface of the patient's skin and between each of the first sensor elements 151. The electronics preferably also include analog-to-digital signal conversion means connected with a signal amplifier for producing digital data representing the patient's ECG waveform over a predefined interval of time. Furthermore, the electronics of micro processor 120 may be equipped with algorithms for identifying ECG events such as QT interval, HRV, heart rate, as well as other physiological parameters recorded with the sensor elements identified above. Preferably, the micro processor 120 may comprise a Digital Signal Processor (DSP) or decision system for analyzing the recorded signals, extracting features of interest, and comparing the extracted features with predefined data representing one or more physiological patient conditions.

Moreover, the device is optionally provided with one or more additional secondary sensor elements 152. These secondary sensor elements 152 may provide detection of additional physiological parameters of the patient, such detection being sensed by the secondary sensor elements arranged either inside housing 101 or on the housing surface adapted for application towards the skin of the patient. Alternatively, or in addition, the secondary sensor elements may be provided by sensor elements arranged externally to the device 100 so that wired or wireless information from sensor elements external to housing 101 are transferred to the device 100. Sensed signals from secondary sensor elements 152 may be analyzed simultaneously as the recorded ECG signals by the micro processor 120 or may be analyzed sequentially.

The optional secondary sensor elements 152 are adapted to detect other signals or conditions which themselves cannot be detected by the first sensing elements 151. Such secondary sensor elements 152 may be adapted to sense physiological data related to the patient, detecting signals such as electric, acoustic or optic signals, i.e. the sensors may be capable of sensing one or more of the following physiological parameters: pulse rate, skin temperature, ambient temperature, oxygen saturation, blood pressure, lung wetness, glucose level, thoracic impedance, ICV pressure, CO₂ content of blood, EEG, mean or peak frequency of the alpha wave, position of patient, movement of body, pH-value, respiration rate, acoustic sounds of the heart picked up by a microphone, etc. The secondary sensor elements 152 may detect any combination of the aforementioned parameters.

The skin impedance measurement may be based on any suitable method known in the art. For example, the skin impedance sensor may comprise a concentric type electrode with an outer passive electrode and an inner electrode, e.g. as disclosed in WO Patent application No. 02/069798. Alternatively, a spectral measurement of impedance may be obtained using the technique described in WO application No. 02/069791.

The skin temperature may be measured by any suitable method known in the art, e.g. by means of a thermistor-based sensor. Temperature measurements may include a temperature sensor situated in the transcutaneous delivery means 113 such as in a hollow needle. Alternatively, a temperature sensor is situated near or in the mounting surface of delivery device 100. Furthermore, the drug delivery device 100 may be adapted to measure ambient temperature.

Embodiments using acoustic sensors as the secondary sensor elements 152 providing simultaneous recording of acoustic signals generated by the heart alongside with the recording of ECG signals may be arranged according to the guidelines of US Patent No. 2004/0167416 and WO Patent application No. 2004/078038.

Respiration rate may be detected by the secondary sensor elements 152 constituting a strain gauge affixed to the skin, a piezoelectric sensing element, or alternatively or in addition be extracted from the recorded ECG signals picked up by the first sensor elements 151.

Body movements of the patient may be detected by motion detectors such as tri-axial accelerometers which also may be used to determine the position of the body, i.e. whether the user is standing up or in lying posture. Parameters such as body posture and the extent of body motion provides valuable information in combination with the sensed ECG signals in order to improve the reliability of the identification procedure of detecting a specific physiological condition. Motion detection or body posture information can be used to distinguish whether a sensed physiological condition corresponds to a high activity level of the user or whether a user is experiencing a glucose crash.

The signals from the secondary sensor elements 152 are, along with signals detected by the first sensor elements 151, forwarded to the central processing unit of micro processor 120 to support the analysis of detected physiological data, and to increase the reliability of the sensed physiological conditions.

An additional electrode (not shown) may be arranged on the upper part of the housing 101 permitting the user to grasp the additional electrode with a hand, and consequently, providing a signal path through the heart region of the user. This arrangement is beneficial if increased reliability and precision is specifically required. The signals picked up by the first sensor elements 151 and, optionally, the secondary sensor elements 152, may be evaluated by the electronic circuitry to decide whether such additional sensor inputs are required.

Manual user inputs, such as accurate glucose readings obtained by invasive measurements, may be entered into the system by way of push-buttons 162 or the like arranged on the housing 101. Additional manual user inputs may be transferred from an external device via wired or wireless communication means (not shown). Said manual inputs may provide data obtained by additional sensors to assist in detecting a specific physiological condition or may be used for calibrating the sensor circuitry. Furthermore, said manual inputs may be used to provide additional information regarding the physical state of the user, such as information on food intake, insulin or glucagon intake or the like. Further, the user inputs may be used to verify, accept or delete a raised alarm regarding a detected physiological condition.

In response to a detected physiological condition within the patient, the drug delivery device 100 may be programmed to perform a change in the rate of drug delivery as a function of the detected physiological condition. Alternatively, or in combination, the drug delivery device 100 may comprise response means 160 for providing a response, such as an audible or visible response to the detected physiological condition, thus providing manual intervention by the user, or alternatively, by a medical adviser. The response means 160 may also transmit a signal to an external device in response to a detected physiological condition.

A non-limiting example of a suitable sensing circuitry which is adapted for incorporation in the drug delivery device according to the invention is shown in the block diagram of Fig. 1 b. A plurality of first sensor elements 151 arranged in the drug delivery device or patch 100 are picking up ECG related electrical signals from the patient carrying the patch 100. Optionally, secondary sensor elements 152 situated in the housing 101 senses various additional data related to other physiological parameters of the patient's body. Also, the secondary sensor elements may comprise sensors 153 external to the housing 101 of drug delivery device 101. The remote sensors 153 are arranged to pick up additional sensor inputs thereby providing signals related to various sensed parameters each of which may be transferred by wired or wireless communication to the delivery device 101. Remote sensors 153 may be external to the body of the patient or may be subcutaneously implanted in the patient.

The measured sensor signals from the first sensor elements 151, and optionally additional data picked up by secondary sensor elements 152 or 153, are forwarded to an input circuit 121, which includes amplifiers and filters for reducing noise and passing the ECG signals along with other physiological data of interest. The output of the input circuitry 121 is fed to an A/D converter 122, which samples and digitizes the sensed signals. The output from the A/D converter 122 is forwarded to a digital signal processor (DSP) 123 which analyzes the individual received parameters and extracts features of interest. Digital signal processor 123 is in communication with memory 124 for controlling the storage of the received sensor signals. Signals received by the digital signal processor 123 may be processed in an internal decision system. According to the decision made by the decision system, output data are generated which are directed to response means 160. According to the output data, the response means 160 controls which action should be taken, i.e. decides whether an alarm should be raised or, alternatively, responds by indicating that further sensor data or manual inputs are required to make a reliable decision. Corresponding to the action taken by the response means, an signal is created via a loudspeaker 163, a display or similar output means.

It is to be noted that the sensing circuitry described above only constitute a single example of various alternatives which falls within the scope of this invention. Other embodiments will be readily apparent to a person skilled in the art, such embodiments including the possibility of designing the drug delivery device as forming a characteristic monitor for detecting and possibly also recording physiological parameters without circuitry for analyzing or decision making. Such a system may instead rely on the transmission of the recorded physiological parameters to an external device 200 performing the necessary steps for analyzing and decision making. The transmission of data to such an external device or remote system 200 may be accomplished at different points along the sensing circuitry as indicated by broken arrows in Fig. 1b. The signal transmission may be provided by any means known in the art, both as one-way communication or as two-way communication. The external device 200 may also be designed as a remote control for user control of the drug delivery device 100. As indicated in fig. 1b, sensor signals from external sensors 153 may be transferred directly to external device 200, or the external device 200 can be provided with integral sensors for measuring additional physiological parameters.

Recording of the different physiological parameters by sensing elements 151, 152 and 153 are performed at regular time intervals either by recording a plurality of different physiological parameters simultaneously or by following a sequential scheme.

In order for the drug delivery device 100 to save energy, the drug delivery device may be designed to record only a single parameter such as the heart rate variability (HRV), or perhaps a limited number of physiological parameters, on a regular basis while other additional parameters are only detected when one or more predefined conditions have been identified by the decision system. On this basis, the system may automatically increase the number of physiological parameters to be included in a more extensive analysis before alerting the user of a potential unsafe condition or before the response means 160 are deemed to sound an alarm.

Also, the sampling rate of the measurements may be automatically adjusted in accordance with the sensed physiological parameters or identified conditions. As long as the decision system detects physiological conditions which correspond to a normal and safe physiological condition of the user, the sampling rate may be low. If the decision system detects an unsafe condition, or possibly, if the detected signals are deemed to be unreliable, the sampling rate may be automatically increased in order to improve the reliability of the detection system. Both systems either consisting of the drug delivery device 100 as a stand-alone apparatus or systems which in addition comprises an external device 200, the power saving arrangements described above will be favorable. When the drug delivery device 100 mainly is designed as a characteristic monitor for sending data to an external device 200, i.e. where the delivery device 100 are designed without extensive computing capabilities, the delivery device may rely on somewhat simple detection schemes which may be used for control of the data rate transmitted to the external device 200.

ECG signals which are picked up at the abdominal region contain several artifacts, such as EMG muscle noise, power line (50Hz) noise, respiratory noise or motion artifacts. Due to these artifacts, preferably two or more first sensor elements 151 are arranged to pick up ECG related signals.

Depending on the activity level of the user, the ECG signals extracted from signals sensed at the abdomen most likely needs to be filtered and preferably averaged over a predetermined number of heart beats in order to obtain a reliable signal. Also, selective averaging may be carried out, i.e. averaging based on recordings where particularly noisy recordings are filtered out before averaging the remaining recordings. In dependence on which ECG signal components or parameters are selected for further analysis, different filtering techniques and detecting algorithms are chosen. Detection algorithms may include various different correlation, autocorrelation and filtering methods.

Optionally, a measure of the signal to noise ratio (SNR) can be calculated in order to obtain an estimate of the quality of the signals which are recorded. From this measure, the SNR can be used to tell when an algorithm becomes useless or to distinguish whether a reliable measurement can be obtained, and thus to decide whether additional sensor inputs or user inputs should be requested.

In one embodiment of the invention, the analysis is based on the heart rate, which preferably is calculated from the RR interval of the ECG. Other embodiments include analysis of T-wave components.

In a further embodiment of the invention, the analysis of the detected ECG signals takes place according to the method described in WO application No. 02/069798. One or more of the physiological parameters which are used to establish the onset or presence of a physiological condition are picked up by first sensor elements 151 arranged in the mounting surface of drug delivery device 100. First sensor elements 151 are adapted to provide Electrocardiogram signals. Optionally, the first sensor elements 151 can be used to detect other bioelectrical signals which correlate to predetermined physiological conditions. Also, the sensor elements 151 may apply an electrical current or voltage to the skin of the patient, whereby the sensing electronics detects a response to the applied signal. By using the same sensor elements to detect several different physiological parameters e.g. by sequential detection of the different physiological parameters, a compact and economical solution is obtained. Additional secondary sensor elements 152 or 153 may be used in order to obtain other physiological parameters which may be used in assisting the detection of an onset or the presence of the physiological condition.

In a still further embodiment of the invention, the analysis of the received ECG signals is processed in accordance with the teaching of co-pending PCT application WO 2005/037092. Also in this embodiment, the first sensor elements 151 are arranged in the mounting surface of drug delivery device 100, optionally with secondary sensor elements 152 arranged within the drug delivery device 100 or provided by external sensing elements 153.

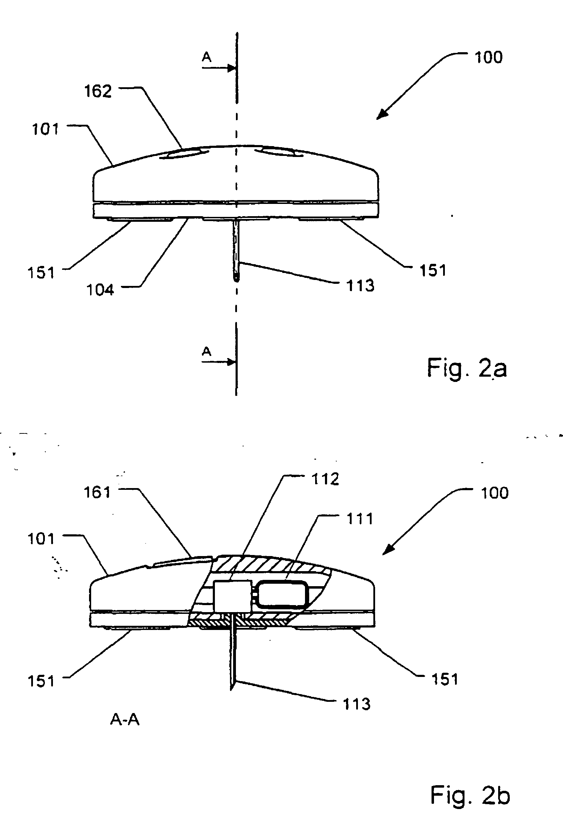

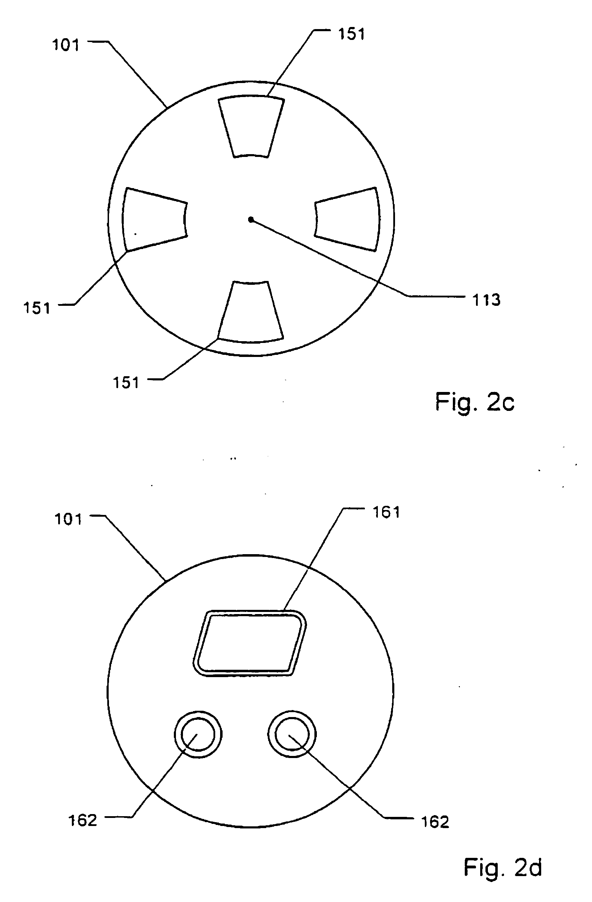

A second embodiment of drug delivery device 100 illustrated in Fig. 2a-2d includes a housing 101 of disc or cylindrical configuration having a flat skin mounting surface 104 coated with a pressure-sensitive adhesive (not shown) for adhering the housing 101 to the skin of the subject to receive the drug. A hollow needle 113 extends through housing 101 through the skin mounting surface 104. The inner end of needle 113 communicates with a pump actuator 112 which expels metered amounts of a drug contained in reservoir 111. The outer end of needle 113 projects outwardly of the skin mounting surface 104 of the housing a short distance so as to penetrate the epidermis of the subject's skin when the housing 101 is applied adhered thereto. The rate and time of delivery of the drug is controlled by internal electrical circuitry (not shown). Housing 101 further comprises a user interface comprising push-buttons 162 and a display 161.

The lower part of housing 101 further comprises two or more first sensor elements 151 in the form of skin electrodes adapted to remain in electrical conductive contact with the skin of the user, when the housing 101 is applied adhered thereto. The first sensor elements 151 are essentially flush with the skin mounting surface 104. In the embodiment shown in fig. 2a-2d, four sensor elements 151 are evenly distributed along the perimeter of a circle which substantially corresponds to the outline of housing 101. Two electrodes will then be able to measure the near-field and the other two electrodes will measure the far-field from the heart dipole. Each of the first sensor elements 151 are electrically connected to electronic sensing circuitry (not shown) arranged in the interior of housing 101. A 4-electrode structure makes a robust sensing scheme with respect to different orientations in the abdominal region.

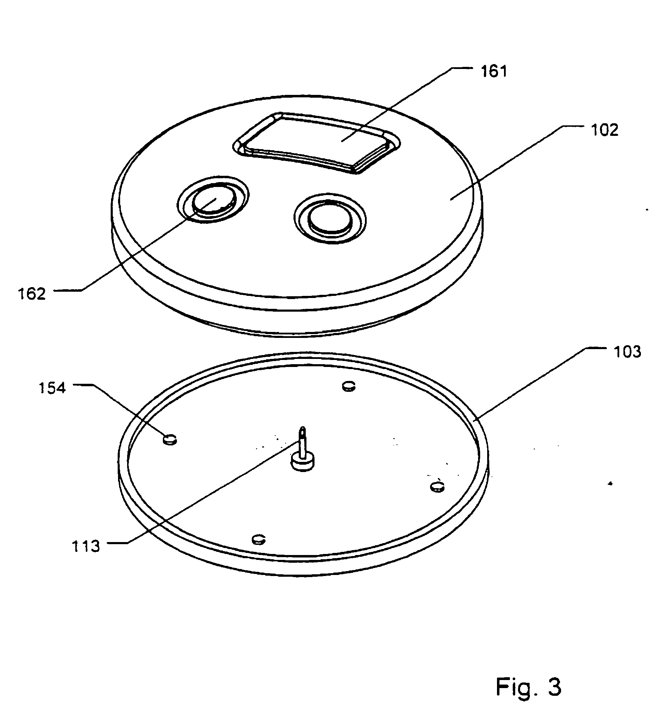

As indicated in the third embodiment depicted on Fig. 3, the drug delivery device 100 may include a disposable assembly 103 having a hollow needle 113, four skin contact electrodes 151 each having an interior part 154 protruding through openings in the disposable assembly 103, a pump actuator (not shown) and a drug reservoir (not shown). This delivery device according to the third embodiment further includes a reusable assembly 102 having a control portion to control the pump actuator upon attachment of the reusable assembly and the disposable assembly, contact points (not shown) for mating with the interior parts 154 of skin contact electrodes 151 and associated circuitry for the sensing of ECG signals (not shown). Reusable assembly further includes a local micro processor 120 connected to the control portion associated with the pump actuator and connected to the sensing circuitry for analyzing the individual received parameters from skin contact electrodes.

A further embodiment (not shown) may comprise a reusable assembly 102 which includes some or all of the sensor elements (151) and/or the secondary sensor elements (152). In this configuration, sensor elements protrudes through openings in the disposable assembly (103) in order to make contact with with the skin of the user, or alternatively, via conductive gel pads or other elements which are able to convey relevant signals or parameters in accordance with the selected sensor parameters.

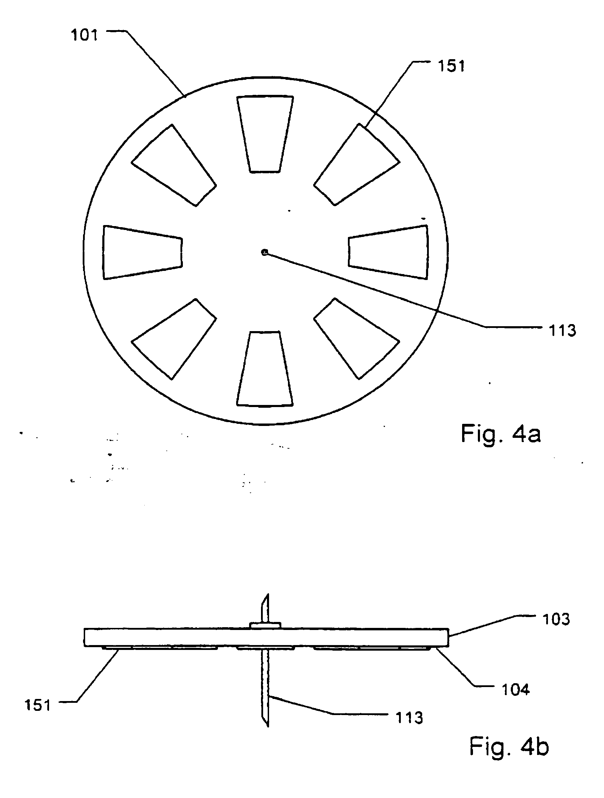

Fig. 4a and 4b illustrates a variation of the disposable assembly of the embodiment shown in fig. 3. Here eight individual skin contact electrodes 151 are evenly distributed along the perimeter of the skin mounting surface 104 of disposable assembly 103. This configuration is less sensitive regarding the quality of the received signals as a function of the angular orientation of the delivery device with respect to a line drawn from the heart region of the patient to the application site of the drug delivery device 100. Also, increasing the number of skin contact electrodes 151 provides increased potential for noise cancellation.

Although the embodiments shown in Fig 2a-d, 3 and 4a-b shows electrode configurations where electrodes are arranged in a circular configuration, it is to be noted that various other layouts of electrode structures including altering the number or electrodes are feasible without parting from the scope of this invention.

Instead of using plural dedicated sensor elements 151 and 152, which are only used for this particular purpose, the invention provides for the possibility of having the transcutaneous access means (which may be a hollow needle 113) constitute one of said sensor elements 151 and 152. The same applies to the skin mounting surface 104 of the housing, which may form one of the sensor elements 151 or 152. In other configurations either the transcutaneous access means 113 or the mounting surface 104 of the housing 101 may define a ground potential.

In order to define a specific area of the skin mounting surface 104 of the housing as an electrical conductive electrode adapted to sense physiological signals, the skin mounting surface 104 may be provided with an electrical conductive adhesive covering this specific area while remaining areas of the skin mounting surface 104 may be provided with non-conductive adhesive. Other exemplary embodiments may comprise a layer of conducting elements known in the art as zebra-connectors arranged between the mounting surface (104) and the skin of the patient for obtaining electrical conductive contact between specific areas of the skin of the patient and corresponding sensor areas of the drug delivery device.

Optimal contact between the sensing elements 151 and 152 and the skin surface of the user may be obtained if the lower part of the housing or alternatively the complete housing 101 is made of resilient material so as to conform to the shape of the skin surface at the application site of delivery device 100. Such a design may be especially beneficial for continuous measuring while the user of drug delivery device 100 is exercising. Also, the sensor material may be somewhat elastic or the mounting of the sensors 151 and 152 may be arranged so as to be able to flex when mechanical forces act upon one or more of the sensors. An adhesive may be used on the mounting surface of each sensor element in order to secure optimal contact between the individual sensor elements and the skin surface.

An alternative embodiment (not shown) of the drug delivery device according to the invention comprises a separate flexible film which forms a basepad on which two or more sensor elements 151 in the form of electrical conductive electrodes are defined. The basepad may be designed according to the teaching of US Patent No. 5,724,984. Electrical connectivity between the electrodes of basepad and the remaining electronics of drug delivery device 100 may be provided by any means known in the art. During use of the drug delivery device, the basepad is situated between mounting surface 104 of housing 101 and the skin surface of the user. Preferably, the basepad substantially corresponds to the outline of the drug delivery device 100. The face of the basepad facing the skin of the user may be provided with an adhesive for attachment to the skin of the user. The opposite face of the basepad may be attached to drug delivery device 100 at various distinct points or lines distributed evenly along the mounting surface 104 or, alternatively, along the entire area of mounting surface 104 of drug delivery device 100. An alternative fixation between drug delivery device 100 and the skin of the user may be accomplished by using a flexible basepad which is provided with cut-outs which mates with adhesive areas on the mounting surface 104. Further, the attachment can be accomplished by having a mounting surface extending beyond the basepad along the perimeter of the mounting surface, and having an adhesive fixation along this perimeter between the mounting surface 104 and the skin. In the last two configurations, the delivery device 100 is directly attached to the skin of the user at the specified adhesive areas on the mounting surface 104. In order to urge the basepad against the skin of the user, a flexible foam cushion or the like may be arranged between the basepad and mounting surface 104.

Also, as an alternative to conventional ECG skin contact electrodes, one or more of the first sensor elements 151 may take the form of a substrate provided with nano spikes similar to the type described in US Patent No. 6,690,959. Such nano spikes shaped to penetrate the epidermis of the skin may be adapted to be incorporated in any of the embodiments of the drug delivery device 100 as described in this application.

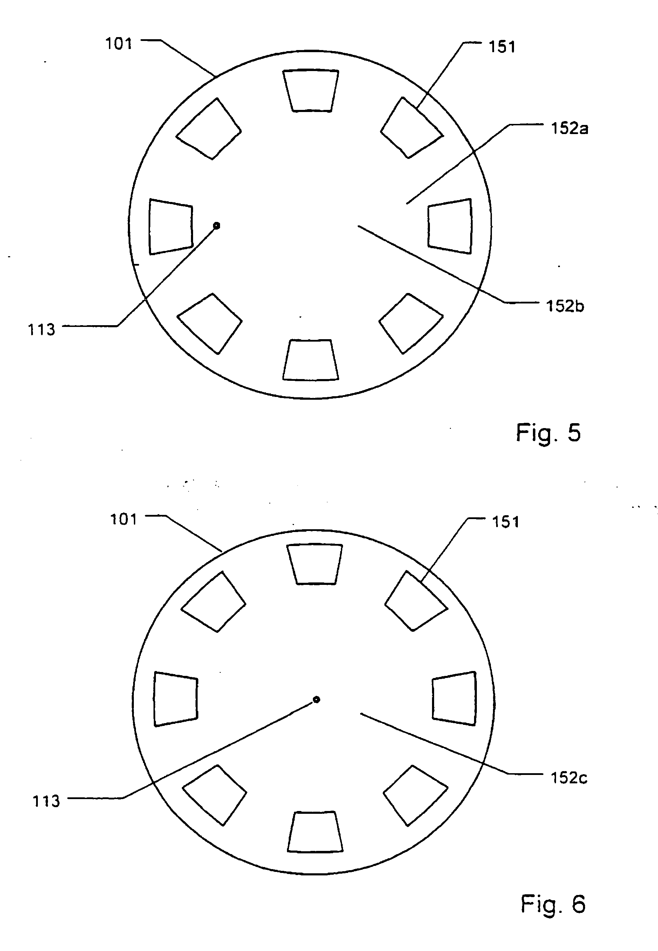

Fig. 5 illustrates a fifth embodiment of the invention, wherein the skin mounting surface 104, like the embodiment shown in fig. 4a, is provided with eight individual skin contact electrodes 151. Furthermore, the skin mounting surface 104 is provided with secondary sensor elements 152. In an exemplary embodiment, a non-invasive measurement of a substance in body fluid, such as the glucose level in blood or tissue, is performed by using radio wave impedance spectroscopy by the technique described in WO Patent application No. 02/069791. In the illustrated embodiment, the secondary sensor elements 152 comprise a ring electrode 152a and a string electrode 152b. The ring electrode 152a is arranged in electrically conductive contact with the skin of the user while string electrode 152b is electrically insulated from the skin of the user.

Fig. 6 illustrates a sixth embodiment of the invention, wherein secondary sensor elements 152 are adapted for measuring skin impedance of the subject user. In this configuration a circular electrode 152c is arranged on the skin mounting surface 104 encircling a hollow needle 113 of the drug delivery device 101. Preferably, a layer of an electrically insulating material is provided along the outer surface of the hollow needle 113 at the end of the needle facing the skin mounting surface 104, while leaving the tip point of the needle exposed to electric interactions. When the drug delivery device is affixed to the user, the tip end of the hollow needle will be in electrical contact with the subdermal tissue and the circular electrode 152c is in electrically conductive contact with the skin of the user. Associated circuitry (not shown) provides the necessary means of recording skin impedance (e.g. DC conductivity, frequency response) and hence provides supplementary physiological parameters for supporting the detection of specific physiological conditions.

Any of the embodiments of the fluid delivery device 100 described in this application may be combined with additional sensor elements 152 or 153 in the form of accelerometers, such as tri-axial accelerometers providing means to distinguish whether or not the user is exercising. Also, the accelerometers may provide information regarding the level or extent of physical activity of the user.

Patterns of body movement may be analyzed to estimate the activity type in order to distinguish whether the body movement is due to external accelerations - e.g. driving a car - or due to physical workout activities. In one embodiment, a feed-forward neural network is utilized for filtering the different patterns of accelerations and linking them to activity types. For most people, external accelerations make an insignificant contribution to the overall amount of acceleration during a typical day. However, for some people, e.g. truck drivers, the contribution from external accelerations needs to be filtered out. The neural network is preferably pre-programmed with typical acceleration patterns and may be configured to adapt and learn from observation, e.g. by using the backpropagation rule. In case the network sees a pattern it cannot classify, it prompts the user to classify the pattern. The inputs to the network may comprise the three acceleration vectors for a number of time steps backwards, time of day, and the previous classified acceleration pattern.

Applications for the accelerometer system which is described above, may include monitoring of drastic accelerations which may occur at severe emergencies like situations where the user has fallen and is unable to get up for instance due to hypoglycemia. Alternatively, or in addition, the body posture of the user may be estimated by body posture sensors.

Various other parameters detected by the remaining sensor elements 151, 152 or 153, e.g. one or more of the parameters obtained by the ECG recordings, such as the heart rate, may be used to distinguish whether the body movements corresponds to physical workout or external accelerations, and hence improve the reliability of the detection of physical activity level. Other non-exhaustive examples of physiological parameters may comprise pulse rate, skin impedance, EMG muscle activity, temperature, etc. Also, the physical activity measures provided by the accelerometer may be used to establish or support the cause for a detected elevated heart rate. Hence, an alarm which has been raised as a possible hypoglycemic state and which is based on certain ECG related parameters, may be filtered out if the activity level based on accelerometer readings do not match the identified physiological condition. Also, it may be estimated whether an elevated heart rate comes from excitement and stress.

The accelerometer readings may furthermore be used for decision support, e.g. when an identified physiological condition based on recorded ECG parameters corresponds to sleep activity of the user. Hence, the identification of sleep activity may be used to modify the injection scheme to correct for glucose level changes due to sleep.

By observing the physical activity level over time, the energy expenditure of the patient can be estimated. The measure of energy expenditure may be used to correct future doses of medication to be injected by the fluid delivery device 100. Also the calculated energy expenditure may be used to warn the user in the event that the user exercises too long and with too high an intensity compared to predetermined physical states of the user. For diabetic patients, this applies in particular when the user exercises too much in comparison to the amount of previously injected insulin.

Any of the embodiments of the fluid delivery device 100 described in this application may be designed to alter the drug delivery as a function of the sensing of an onset or the presence of an identified physiological condition. Ultimately, the system is in the form of a closed loop system adapted for controlling the physiological condition of a patient by infusing a drug as a response to a sensor signal indicative of one or more physiological conditions.

As described in US Patent No. 5,741,211, ECG related signals can be used for determining a measure of glucose concentration in blood, thereby obtaining a feedback loop for controlling drug infusion. According to the present invention, providing ECG sensing elements 151 in the same housing as the drug delivery device 100, it is possible to obtain a self-contained drug delivery device 100 with integrated ECG sensor elements 151. Such a system may comprise control means 120 adapted to receive the signals from the sensor system (derived from analyzed ECG signals) and generate command signals in response thereto in order to keep the blood glucose level of the patient within a desired range, and delivery means for delivering an amount of at least one drug having a blood glucose regulating effect, wherein operation of the delivery means is affected by the command signals.

One further embodiment of a drug delivery device comprises an additional fluid reservoir (not shown) containing a fast acting drug, such as glucagon, being administered in place of the primary drug, which may be insulin. This fast acting drug may be expelled either through the same transcutaneous access means as the primary drug or through dedicated transcutaneous access means, the fluid path being established when a predetermined physiological conditions has been identified. Such a configuration may be used in conjunction with all other embodiments described in this application.

The sensed physiological parameters or the sensed physiological conditions may be indicated on display 161 or be downloaded to an external device 200 such as a PDA or a personal computer for further data analysis or event logging.

Preferably, the drug delivery device may be programmed by an external device such as a personal computer for updating diagnostic software and/or adapting the software/firmware to suit the particular user of the drug delivery device.

Sensed physiological conditions may, alternatively or in addition, be signaled by an audible alert. Audible alerts may be a simple alarm generated by a tone generator or in the form of synthesized or sampled speech providing instructions to the patient or to another person nearby.

Other alarms may comprise vibratory elements or electro-muscle stimulation (EMS), the latter being described in WO Patent application No. 2004/030727. A particularly simple embodiment uses EMS which is applied to the skin of the user by means of the same electrodes as the ones used for sensing the physiological conditions of the patient, i.e. sensor elements 151 or 152.

Still other response means 160 may include an alarm in the form of a signal transmitted to a monitoring station (either a remote emergency center or a monitoring device used by family members), or a signal transmitted via a secondary device such as a cell phone or PDA which forwards the alarm to a central monitoring station. Also, the alarm of the drug delivery device can be adapted to communicate with repeater units or hotspots distributed in the whereabouts of the patient.

Also, any of the above described alarms may be used to signal a possible defect or malfunction of the device itself, such conditions being determined by sensors situated inside the delivery device 100. An example of one operating anomaly or defect is occlusion in the fluid path which is sensed by occlusion sensors in the fluid path. Other examples may include the sensing of the amount of medication remaining in drug reservoir 111. Furthermore, the micro processor 120 may be designed to perform a self check at regular intervals or by user activation, this self check providing an alarm if a defect or malfunction has been identified. Preferably, the alarm for signaling a physiological condition is chosen to be easily distinguished from other alarms signaling malfunctions of the device. The device may also be designed to provide a signal at regular intervals to indicate that the device is working properly, or to indicate that the physiological conditions which are detected are within predefined limits.

Although only a few exemplary embodiments of the present invention have been described in detail above, those skilled in the art will appreciate readily that many modifications are possible in the exemplary embodiments without materially departing from the novel teachings and advantages of the invention. Accordingly, all such modifications are intended to be included within the scope of the present invention as defined in the following claims. For example, micro processor 120 of the present invention may include any one or more of, but is not limited to, a CPU, a processor, a microprocessor, a controller, a microcontroller, an application specific integrated circuit (an ASIC), a digital signal processor (a DSP), a signal conditioning amplifier, pre-amplifier or filter, a micro-computer, a computer and the like. Likewise, the sensor elements 151 and/or 152 may comprise converters for converting a measured analog signal into digital representation before forwarding the measured signals to the micro processor 120.

In the above description of the exemplary embodiments, the different structures providing the desired relations between the different components just as the means providing the described functionality for the different components (processor means, transmitting and receiving means, memory and timer means) have been described to a degree to which the concept of the present invention will be apparent to the skilled reader. The detailed construction and specification for the different structures are considered the object of a normal design procedure performed by the skilled person along the lines set out in the present specification.

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. The citation of patent documents herein is done for convenience only and does not reflect any view of the validity, patentability, and/or enforceability of such patent documents.

This invention includes all modifications and equivalents of the subject matter recited in the claims appended hereto as permitted by applicable law.

## Claims

1. A fluid delivery device (100) for attachment to the skin of a subject user, the delivery device comprising:
- a housing,
- a mounting surface (104) adapted for application to the skin of the subject,
- a reservoir (111) adapted to contain a fluid,
- transcutaneous access means (113) communicating, in a situation of use, with the interior of the reservoir (111) and adapted to penetrate the skin of the subject,
- expelling means (112) for expelling a fluid out of the reservoir (111) through the transcutaneous access means (113),
- first sensor elements (151) adapted for obtaining electric signals representative of ECG signals of said subject, and
- electronic circuitry coupled to said first sensor elements (151) and adapted to measure, monitor, calculate, and/or store data related to said sensed ECG signals, **characterized in that**
- the fluid delivery device is in the form of a single portable item,
- the first sensor elements (151) are disposed on or in said housing,
- the mounting surface (104) comprises adhesive means allowing the device to be affixed to the skin of the subject user, and
- the transcutaneous access means is in the form of a flexible infusion cannula (113), the delivery device thereby being adapted for placement at the abdomen of the subject user.

2. A fluid delivery device as in claim 1, wherein the transcutaneous access means is arranged to penetrate the skin of the subject in an area corresponding to the mounting surface.

3. A fluid delivery device as in claim 1, wherein said first sensor elements (151) comprises sensors capable of sensing one or more physiological parameters selected from the group consisting of heart rate, heart rate variability (HRV), QT interval, QT dispersion, polarity of T wave, amplitude of the T-wave, T-wave ratio, QRS amplitude, QRS length and respiration rate.

4. A fluid delivery device as in any of the previous claims, wherein said first sensor elements (151) are further adapted to sense physiological parameters selected from the group consisting of skin resistance, skin impedance and electromyogram (EMG).

5. A fluid delivery device as in any of the previous claims, wherein the delivery device further comprises secondary sensor elements (152) capable of sensing physiological parameters selected from the group consisting of skin temperature, ambient temperature, oxygen saturation, blood pressure, pulse rate, respiration rate, glucose level, CO₂ content of blood, position of patient, movement of body, acoustic sounds produced by the heart and pH.

6. A fluid delivery device as in any of the previous claims, wherein the delivery device further comprises communication means for receiving physiological parameters detected and transmitted by an external device (153, 200), the external device comprising sensors capable of sensing parameters selected from the group consisting of skin temperature, ambient temperature, ECG, EEG, mean or peak frequency of the alpha wave, oxygen saturation, blood pressure, lung wetness, glucose level, thoracic impedance, ICV pressure, CO₂ content of blood, position of patient, movement of body, acoustic sounds produced by the heart and pH.

7. A fluid delivery device as in any of the previous claims, further comprising means for storing data related to sensor signals obtained by the first sensor elements (151).

8. A fluid delivery device as in any of claims 5-7, further comprising means for storing data related to sensor signals obtained by the secondary sensor elements (152, 153).

9. A fluid delivery device as in any of the previous claims, further comprising processing means (120) for processing said sensed signals to provide data indicative of the presence or onset of a physiological condition of said subject.

10. A fluid delivery device as in claim 9, wherein the physiological condition is selected from the group consisting of hypoglycemia, hyperglycemia, level of blood glucose, physical activity level and sleep activity.

11. A fluid delivery device as in any of the previous claims, further comprising means for transmitting data related to said sensed signals to an external device (200).

12. A fluid delivery device as in any of the previous claims, wherein the first sensor elements (151) are adapted for obtaining native electrical signals not provoked by subcutaneously implanted devices.

13. A fluid delivery device as in any of the previous claims, wherein the first sensor elements (151) comprises two or more electrodes adapted for contacting the skin of said subject.

14. A fluid delivery device as in any of the previous claims, wherein said transcutaneous access means (113) constitutes one of said first sensor elements (151).

15. A fluid delivery device as in any of the previous claims, further comprising a mounting surface (104) adapted for application to the skin of the subject, wherein the mounting surface (104) of said fluid delivery device (100) constitutes one of said first sensor elements (151).

16. A fluid delivery device as in any the previous claims, wherein the fluid is selected from the group consisting of insulin and glucagons.

17. A fluid delivery device as in any of claims 9-16, wherein the delivery device comprises response means (160) for providing a response as a function of an identified presence or onset of a physiological condition of said subject.

18. A fluid delivery device as in claim 17, wherein the response means (160) comprises output means (161,163) providing signals selected from the group consisting of audible signals, visual signals, tactile signals, electro-muscle stimulation and vibratory signals.

19. A fluid delivery device as in claims 17 or 18, wherein the expelling means (112) are controlled by a control signal, and that said control signal is based at least in part on the response taken by the response means (160).

20. A fluid delivery device as in any the previous claims, wherein one or more of the first sensor elements (151) are provided with an electrical conducting adhesive for attachment to the skin of the user.

21. A system comprising a fluid delivery device (100) as in any of claims 1-20, and further comprising an external device (200) separate from said fluid delivery device (100), wherein:
- said fluid delivery device (100) comprises communication means for communicating with said external device (200),
- said external device (200) comprises communication means adapted for communicating with the fluid delivery device (100) such that information related to sensor signals obtained by the delivery device (100) can be transferred to the external device (200).

22. A system as in claim 21, wherein said transferred information comprises partial or full representations of sensed ECG signals.

23. A system as in claims 21 or 22, wherein said transferred information comprises extracted features of sensed ECG signals.

24. A system as defined in any of claims 21-23, wherein the external device (200) further comprises processing means for processing said transferred information to provide data indicative of the presence or onset of a physiological condition of said subject.

25. A system as in claim 26, wherein said external device (200) comprises means for presenting an output as a response to an identified presence or onset of a physiological condition of said subject.

26. A system as in claims 24 or 25, wherein said external device (200) further comprises means for transferring information related to an identified presence or onset of a physiological condition of said subject to said fluid delivery device (100).

## Patentansprüche

1. Fluidabgabevorrichtung (100) zum Anhaften an die Haut eines Anwenders, wobei die Abgabevorrichtung umfasst:
- ein Gehäuse,
- eine Befestigungsfläche (104), die zum Aufbringen auf die Haut des Anwenders angepasst ist,
- einen Behälter (111), der zum Aufnehmen eines Fluids angepasst ist,
- ein Mittel zum transkutanen Zugang (113), das in Verwendungssituation mit dem Inneren des Behälters (111) kommuniziert und zum Durchdringen der Haut des Anwenders angepasst ist,
- ein Ausstoßmittel (112) zum Ausstoßen eines Fluids aus dem Behälter (111) durch das Mittel zum transkutanen Zugang (113),
- erste Sensorelemente (151), die zum Erhalt von für EKG-Signale des Anwenders repräsentativen elektrischen Signalen angepasst sind, und
- einen elektronischen Schaltkreis, der an die ersten Sensorelemente (151) gekoppelt und zum Messen, Überwachen, Berechnen und/oder Speichern von Daten in Bezug auf die ertasteten EKG-Signale angepasst ist,
**dadurch gekennzeichnet, dass**
- die Fluidabgabevorrichtung in der Form eines einzelnen tragbaren Gegenstandes vorliegt,
- die ersten Sensorelemente (151) auf oder in dem Gehäuse angeordnet sind,
- die Befestigungsfläche (104) Haftmittel umfasst, die es der Vorrichtung erlauben, an der Haut des Anwenders befestigt zu werden, und
- das Mittel zum transkutanen Zugang in der Form einer flexiblen Infusionskanüle (113) vorliegt, wobei die Abgabevorrichtung **dadurch** zum Ansetzen an den Bauch des Anwenders angepasst ist.

2. Fluidabgabevorrichtung nach Anspruch 1, wobei das Mittel zum transkutanen Zugang angeordnet ist, die Haut des Anwenders in einer der Befestigungsfläche entsprechenden Fläche zu durchdringen.

3. Fluidabgabevorrichtung nach Anspruch 1, wobei die ersten Sensorelemente (151) Sensoren umfassen, die in der Lage sind, einen oder mehrere physiologische Parameter, ausgewählt aus der Gruppe, bestehend aus Herzfrequenz, Herzfrequenzvariabilität (heart rate variability; HRV), QT-Intervall, QT-Streuung, T-Wellenpolarität, T-Wellenamplitude, T-Wellenverhältnis, CRS-Amplitude, QRS-Länge und Atemfrequenz, zu ertasten.

4. Fluidabgabevorrichtung nach einem der vorangehenden Ansprüche, wobei die ersten Sensorelemente (151) des Weiteren angepasst sind, physiologische Parameter, ausgewählt aus der Gruppe, bestehend aus Hautwiderstand, Hautimpedanz und Elektromyogramm (EMG) zu ertasten.

5. Fluidabgabevorrichtung nach einem der vorangehenden Ansprüche, wobei die Abgabevorrichtung des Weiteren zweite Sensorelemente (152) umfasst, die in der Lage sind, physiologische Parameter, ausgewählt aus der Gruppe, bestehend aus Hauttemperatur, Umgebungstemperatur, Sauerstoffsättigung, Blutdruck, Pulsfrequenz, Atemfrequenz, Glukosespiegel, CO₂-Gehalt des Bluts, Patientenposition, Körperbewegung, durch das Herz erzeugten akustischen Töne und pH-Wert, zu ertasten.

6. Fluidabgabevorrichtung nach einem der vorangehenden Ansprüche, wobei die Abgabevorrichtung des Weiteren Kommunikationsmittel zum Empfangen von durch eine externe Vorrichtung (153, 200) erfassten und gesendeten Parametern umfasst, wobei die externe Vorrichtung Sensoren umfasst, die in der Lage sind, Parameter, ausgewählt aus der Gruppe, bestehend aus Hauttemperatur, Umgebungstemperatur, EKG, EEG, Mittel- oder Spitzenfrequenz der Alphawelle, Sauerstoffsättigung, Blutdruck, Lungenfeuchtigkeit, Glukosespiegel, Thoraximpedanz, ICV-Druck, CO₂-Gehalt des Bluts, Patientenposition, Körperbewegung, durch das Herz erzeugte akustische Töne und pH-Wert, zu ertasten.

7. Fluidabgabevorrichtung nach einem der vorangehenden Ansprüche, des Weiteren umfassend Mittel zum Speichern von Daten in Bezug auf durch die ersten Sensorelemente (151) erhaltene Sensorsignale.

8. Fluidabgabevorrichtung nach einem der Ansprüche 5-7, des Weiteren umfassend Mittel zum Speichern von Daten in Bezug auf durch die zweiten Sensorelemente (152, 153) erhaltene Sensorsignale.

9. Fluidabgabevorrichtung nach einem der vorangehenden Ansprüche, des Weiteren umfassend Verarbeitungsmittel (120) zum Verarbeiten der ertasteten Signale zum Bereitstellen von Daten, die auf die Gegenwart oder den Einsatz eines physiologischen Zustands des Anwenders hinweisen.

10. Fluidabgabevorrichtung nach Anspruch 9, wobei der physiologische Zustand ausgewählt ist aus der Gruppe, bestehend aus Hypoglykämie, Hyperglykämie, Blutglukosespiegel, Grad an physikalischer Aktivität und Schlafaktivität.

11. Fluidabgabevorrichtung nach einem der vorangehenden Ansprüche, des Weiteren umfassend Mittel zum Senden von Daten in Bezug auf die ertasteten Signale an eine externe Vorrichtung (200).

12. Fluidabgabevorrichtung nach einem der vorangehenden Ansprüche, wobei die ersten Sensorelemente (151) zum Erhalt von natürlichen Signalen angepasst sind, die nicht durch subkutan implantierte Vorrichtungen hervorgerufen werden.

13. Fluidabgabevorrichtung nach einem der vorangehenden Ansprüche, wobei die ersten Sensorelemente (151) zwei oder mehr Elektroden umfassen, die zum Inkontaktkommen mit der Haut des Anwenders angepasst sind.

14. Fluidabgabevorrichtung nach einem der vorangehenden Ansprüche, wobei das Mittel zum transkutanen Zugang (113) eines der ersten Sensorelemente (151) bildet.

15. Fluidabgabevorrichtung nach einem der vorangehenden Ansprüche, des Weiteren umfassend eine Befestigungsfläche (104) die zum Aufbringen auf die Haut des Anwenders angepasst ist, wobei die Befestigungsfläche (104) der Fluidabgabevorrichtung (100) eines der ersten Sensorelemente (151) bildet.

16. Fluidabgabevorrichtung nach einem der vorangehenden Ansprüche, wobei das Fluid ausgewählt ist aus der Gruppe, bestehend aus Insulin und Glucagonen.

17. Fluidabgabevorrichtung nach einem der Ansprüche 9-16, wobei die Abgabevorrichtung ein Antwortmittel (160) zum Bereitstellen einer Antwort als Funktion einer erkannten Gegenwart oder eines erkannten Einsatzes eines physiologischen Zustands des Anwenders umfasst.

18. Fluidabgabevorrichtung nach Anspruch 17, wobei das Antwortmittel (160) Ausgabemittel (161, 163) umfasst, die Signale, ausgewählt aus der Gruppe, bestehend aus akustischen Signalen, visuellen Signalen, tastbaren Signalen, Elektromuskelstimulation und Vibrationssignalen, bereitstellen.

19. Fluidabgabevorrichtung nach den Ansprüchen 17 oder 18, wobei die Ausstoßmittel (112) durch ein Steuersignal gesteuert werden und das Steuersignal zumindest teilweise auf der durch das Antwortmittel (160) aufgenommenen Antwort basiert.

20. Fluidabgabevorrichtung nach einem der vorangehenden Ansprüche, wobei ein oder mehrere der ersten Sensorelemente (151) mit einem elektrisch leitenden Haftmittel zum Aufbringen auf die Haut des Anwenders versehen sind.

21. System, umfassend eine Fluidabgabevorrichtung (100) nach den Ansprüchen 1-20 und des Weiteren eine von der Fluidabgabevorrichtung (100) getrennte externe Vorrichtung (200), wobei
- die Fluidabgabevorrichtung (100) Kommunikationsmittel zum Kommunizieren mit der externen Vorrichtung (200) umfasst,
- wobei die externe Vorrichtung (200) Kommunikationsmittel umfasst, die zum Kommunizieren mit der Fluidabgabevorrichtung (100) angepasst sind, sodass Informationen in Bezug auf durch die Abgabevorrichtung (100) erhaltene Sensorsignale zu der externen Vorrichtung (200) übertragen werden können.

22. System nach Anspruch 21, wobei die übertragenen Informationen teilweise oder vollständige Repräsentationen von ertasteten EKG-Signalen umfassen.

23. System nach Anspruch 21 oder 22, wobei die übertragenen Informationen extrahierte Merkmale von ertasteten EKG-Signalen umfassen.

24. System nach einem der Ansprüche 21-23, wobei die externe Vorrichtung (200) des Weiteren Verarbeitungsmittel zum Verarbeiten der übertragenen Informationen umfasst, um Daten bereitzustellen, die auf die Gegenwart oder den Einsatz eines physiologischen Zustands des Anwenders hinweisen.

25. System nach Anspruch 26, wobei die externe Vorrichtung (200) Mittel zum Darstellen einer Ausgabe als Antwort auf eine erkannte Gegenwart oder einen erkannten Einsatz eines physiologischen Zustands des Anwenders umfasst.

26. System nach den Ansprüchen 24 oder 25, wobei die externe Vorrichtung (200) Mittel zum Übertragen von Informationen in Bezug auf eine erkannte Gegenwart oder einen erkannten Einsatz eines physiologischen Zustands des Anwenders zu der Fluidabgabevorrichtung (100) umfasst.

## Revendications

1. Un dispositif de délivrance de fluide (100) destiné à être solidarisé à la peau d'un sujet utilisateur, le dispositif de délivrance comprenant :
- un boîtier,
- une surface de montage (104) apte à être appliquée sur la peau du sujet,
- un réservoir (111) apte à contenir un fluide,
- des moyens d'accès transcutané (113) communiquant, dans une situation d'utilisation, avec l'intérieur du réservoir (111) et aptes à pénétrer dans la peau du sujet,
- des moyens d'expulsion (112) pour expulser un fluide hors du réservoir (111) par l'intermédiaire des moyens d'accès transcutané (113),
- des premiers éléments capteurs (151) aptes à obtenir des signaux électriques représentatifs de signaux ECG dudit sujet, et
- un ensemble de circuits électroniques couplé auxdits premiers éléments capteurs (151) et apte à mesurer, surveiller, calculer et/ou stocker des données en relation avec lesdits signaux ECG captés, **caractérisé en ce que**
- le dispositif de délivrance de fluide est sous forme d'un élément portatif unique,
- les premiers éléments capteurs (151) sont disposés sur ledit boîtier ou dans celui-ci,
- la surface de montage (104) comprend des moyens adhésifs permettant au dispositif d'être fixé sur la peau du sujet utilisateur, et
- les moyens d'accès transcutané sont sous forme d'une canule de perfusion flexible (113), le dispositif de délivrance étant ainsi apte à être placé sur l'abdomen du sujet utilisateur.

2. Un dispositif de délivrance de fluide selon la revendication 1, dans lequel les moyens d'accès transcutané sont configurés pour pénétrer dans la peau du sujet dans une zone correspondant à la surface de montage.

3. Un dispositif de délivrance de fluide selon la revendication 1, dans lequel lesdits premiers éléments capteurs (151) comprennent des capteurs capables de détecter un ou plusieurs paramètres physiologiques choisis dans le groupe constitué par le rythme cardiaque, la variabilité du rythme cardiaque (HRV), l'intervalle QT, la dispersion QT, la polarité de l'onde T, l'amplitude de l'onde T, le ratio de l'onde T, l'amplitude du QRS, la longueur du QRS et la fréquence respiratoire.

4. Un dispositif de délivrance de fluide selon l'une des revendications précédentes, dans lequel lesdits premiers éléments capteurs (151) sont en outre aptes à détecter des paramètres physiologiques choisis dans le groupe constitué par la résistance de peau, l'impédance de peau et un électromyogramme (EMG).

5. Un dispositif de délivrance de fluide selon l'une des revendications précédentes, dans lequel le dispositif de délivrance comprend en outre des éléments capteurs secondaires (152) capables de détecter des paramètres physiologiques choisis dans le groupe constitué par la température de la peau, la température ambiante, la saturation en oxygène, la pression sanguine, le rythme du pouls, la fréquence respiratoire, le niveau de glucose, la teneur en CO₂ du sang, la position du patient, le mouvement du corps, les bruits acoustiques produits par la coeur et le pH.

6. Un dispositif de délivrance de fluide selon l'une des revendications précédentes, dans lequel le dispositif de délivrance comprend en outre des moyens de communication pour recevoir des paramètres physiologiques détectés et émis par un dispositif externe (153, 200), le dispositif externe comprenant des capteurs capables de détecter des paramètres choisis dans le groupe constitué par la température de la peau, la température ambiante, l'ECG, l'EEG, la fréquence moyenne ou de crête de l'onde alpha, la saturation en oxygène, la pression sanguine, l'humidité des poumons, le niveau de glucose, l'impédance thoracique, la pression iCV, la teneur en CO₂ du sang, la position du patient, le mouvement du corps, les bruits acoustiques produits par le coeur et le pH.

7. Un dispositif de délivrance de fluide selon l'une des revendications précédentes, comprenant en outre des moyens pour stocker des données relatives à des signaux de capteur obtenus par les premiers éléments capteurs (151).

8. Un dispositif de délivrance de fluide selon l'une des revendications 5 à 7, comprenant en outre des moyens pour stocker des données relatives aux signaux de capteur obtenus par les éléments capteurs secondaires (152, 153).

9. Un dispositif de délivrance de fluide selon l'une des revendications précédentes, comprenant en outre des moyens de traitement (120) pour traiter lesdits signaux captés pour produire des données représentatives de la présence ou de l'apparition d'un état physiologique dudit sujet.

10. Un dispositif de délivrance de fluide selon la revendication 9, dans lequel l'état physiologique est choisi dans le groupe constitué par l'hypoglycémie, l'hyperglycémie, le niveau de glucose sanguin, le niveau d'activité physique et l'activité de sommeil.

11. Un dispositif de délivrance de fluide selon l'une des revendications précédentes, comprenant en outre des moyens pour émettre des données en relation avec lesdits signaux captés vers un dispositif externe (200).

12. Un dispositif de délivrance de fluide selon l'une des revendications précédentes, dans lequel les premiers éléments capteurs (151) sont aptes à recueillir des signaux électriques natifs non provoqués par des dispositifs implantés sous-cutanés.

13. Un dispositif de délivrance de fluide selon l'une des revendications précédentes, dans lequel les premiers éléments capteurs (151) comprennent deux électrodes ou plus aptes à venir en contact avec la peau dudit sujet.

14. Un dispositif de délivrance de fluide selon l'une des revendications précédentes, dans lequel lesdits moyens d'accès transcutané (113) constituent l'un desdits premiers éléments capteurs (151).

15. Un dispositif de délivrance de fluide selon l'une des revendications précédentes, comprenant en outre une surface de montage (104) apte à être appliquée sur la peau du sujet, dans lequel la surface de montage (104) dudit dispositif de délivrance de fluide (100) constitue l'un desdits premiers éléments capteurs (151).

16. Un dispositif de délivrance de fluide selon l'une des revendications précédentes, dans lequel le fluide est choisi dans le groupe constitué par l'insuline et les glucagons.

17. Un dispositif de délivrance de fluide selon l'une des revendications 9 à 16, dans lequel le dispositif de délivrance comprend des moyens de réponse (160) pour produire une réponse en fonction d'une présence identifiée ou de l'apparition d'un état physiologique dudit sujet.

18. Un dispositif de délivrance de fluide selon la revendication 17, dans lequel les moyens de réponse (160) comprennent des moyens de sortie (161, 163) produisant des signaux choisis dans le groupe constitué par les signaux audibles, les signaux visuels, les signaux tactiles, la stimulation électromusculaire et les signaux vibrants.

19. Un dispositif de délivrance de fluide selon la revendication 17 ou 18, dans lequel les moyens d'expulsion (112) sont contrôlés par un signal de contrôle, et où ledit signal de contrôle est basé au moins en partie sur la réponse donnée par les moyens de réponse (160).

20. Un dispositif de délivrance de fluide selon l'une des revendications précédentes, dans lequel un ou plusieurs des premiers éléments capteurs (151) est prévu avec un adhésif électriquement conducteur pour fixation à la peau de l'utilisateur.

21. Un système comprenant un dispositif de délivrance de fluide (100) selon l'une des revendications 1 à 20, et comprenant en outre un dispositif externe (200) distinct dudit dispositif de délivrance de fluide (100), dans lequel :
- ledit dispositif de délivrance de fluide (100) comprend des moyens de communication pour communiquer avec ledit dispositif externe (200),
- ledit dispositif externe (200) comprend des moyens de communication aptes à communiquer avec le dispositif de délivrance de fluide (100) de sorte que les informations en relation avec les signaux de capteur obtenus par le dispositif de délivrance de fluide (100) puissent être transférés au dispositif externe (200).

22. Un système selon la revendication 21, dans lequel lesdites informations transférées comprennent des représentations partielles ou totales des signaux ECG détectés.

23. Un système selon les revendications 21 ou 22, dans lequel lesdites informations transférées comprennent des caractéristiques extraites des signaux ECG détectés.

24. Un système selon l'une des revendications 21 à 23, dans lequel le dispositif externe (200) comprend en outre des moyens de traitement pour traiter lesdites informations transférées pour produire des données représentatives de la présence ou de l'apparition d'un état physiologique dudit sujet.

25. Un système selon la revendication 26, dans lequel ledit dispositif externe (200) comprend des moyens pour présenter une sortie en tant que réponse à une présence identifiée ou une apparition d'un état physiologique dudit sujet.

26. Un système selon la revendication 24 ou 25, dans lequel ledit dispositif externe (200) comprend en outre des moyens pour transférer des informations en relation avec une présence identifiée ou une apparition d'un état physiologique dudit sujet vers ledit dispositif de délivrance de fluide (100).
